# EUROPEAN PATENT SPECIFICATION

(11) **EP 2 687 247 B1**
(45) Date of publication and mention of the grant of the patent: **16.12.2015**
(21) Application number: 12757743.5
(22) Date of filing: 15.02.2012
(51) Int. Cl.: A61M 1/14, A61M 1/16, A61M 1/36

(54) **HEMODIALYSIS DEVICE**
HÄMODIALYSATOR
DISPOSITIF D'HÉMODIALYSE

(30) Priority: 15.03.2011 JP 2011056611
(43) Date of publication of application: 22.01.2014
(73) Proprietor: Shibuya Kogyo Co., Ltd., Ishikawa 920-8681 (JP)
(72) Inventor: NOZATO Nobuyuki, Kanazawa-shi Ishikawa 920-8681 (JP)
(74) Representative: Hodsdon, Stephen James
(86) International application number: PCT/JP2012/053485
(87) International publication number: WO 2012/124425

(56) References cited:
- DE-A1- 3 442 744
- JP-A- 3 057 458
- JP-A- 61 203 973
- JP-A- 2002 126 076
- JP-A- 2003 093 851
- US-B1- 6 280 632

## Description

### Technical Field

The present invention relates to a hemodialysis apparatus, and in particular, to a hemodialysis apparatus provided with a dialysate filter provided in a dialysate circuit and having an inside divided by a filtration film purifying the dialysate into a space on the inflow side and a space on the discharge side of the dialysate.

### Background Art

As a conventional hemodialysis apparatus, a device provided with a dialyzer that performs hemodialysis and a dialysate circuit composed of a dialysate supply passage for supplying fresh dialysate to the dialyzer and a dialysate recovery passage for recovering used dialysate having passed through the dialyzer has been known.

In such a hemodialysis apparatus, in order to remove endotoxin and the like in the dialysate to be used, a dialysate filter having the inside divided by a filtration film purifying the dialysate into a space on the inflow side and a space on the discharge side of the dialysate is provided, and this dialysate filter is subjected to leak check in advance when dialysis treatment is to be conducted.

When the above described leak check is to be conducted, a differential pressure is generated between one of the spaces and the other in the dialysate filter, and in order to generate such differential pressure, those using a dialysate pump which is provided in the dialysate recovery passage and feeds the used dialysate (Patent Literature 1) and those using draining means for drainage by bringing the dialysate circuit into a negative pressure (Patent Literature 2) are known.

Furthermore apparatus and methods for providing sterile substitution solutions from a dialysis fluid used in connection with the treatment of a patient's blood in an extracorporeal circuit are disclosed (Patent Literature 3). The apparatus includes pumps or valves for comparing the flow of fresh dialysis fluid prior to treatment and after treatment of a patient's blood and a throttle which divides the fresh dialysis circuit into a positive pressure portion and a negative pressure portion, a sterile filter including a membrane disposed in the positive pressure portion of the dialysis circuit, and a valve for controlling the pressure in the positive pressure portion to pass a predetermined amount of the fresh dialysis fluid through the membrane in a sterile filter to provide a predetermined amount of the sterile substitution solution for addition to the extracorporeal circuit.

### Prior Art Documents

### Patent Literature

Patent Literature 1: Japanese Patent No. 4267197
Patent Literature 2: Japanese Patent Publication No. 5-80234
Patent Literaure 3: US Patent No. 6280632 B1

### Summary of Invention

### Problems to be Solved by the Invention

However, the dialysate pump according to the above described Patent Literature 1 has a problem that, though a flow rate thereof is large, a high negative pressure cannot be generated and thus, a negative pressure which is large enough for checking the dialysate filter cannot be obtained.

Moreover, the draining means according to Patent Literature 2 has a problem that, though a high negative pressure can be generated, a fluid feeding amount is small and thus, it takes time to obtain a negative pressure required for inspection, and inspection time might be long.

In view of the above problems, the present invention has an object to provide a hemodialysis apparatus which can conduct leak check of the dialysate filter accurately and rapidly.

### Means for Solving the Problems

Specifically, a hemodialysis apparatus according to claim 1 includes a dialyzer which performs hemodialysis, a dialysate circuit composed of a dialysate supply passage for supplying fresh dialysate to the dialyzer and a dialysate recovery passage for recovering used dialysate having passed through the dialyzer, a dialysate pump provided in the dialysate recovery passage and feeding the used dialysate, draining means for drainage from blood through the dialyzer, a dialysate filter having an inside divided by a filtration film into a space on the inflow side and a space on the discharge side and purifying and discharging the fresh dialysate flowing from the dialysate supply passage, and control means for controlling operations of the dialysate pump and the draining means, comprising:
an inspection passage for connecting one of the space on the inflow side or the space on the discharge side of the dialysate filter to the dialysate recovery passage; opening means for opening the other space on the side opposite to the one space of the dialysate filter into the air; pressure detecting means for detecting pressure fluctuation in the one space; and judgment means for good or bad judgment of the dialysate filter on the basis of a detection result of the pressure detecting means, wherein
when good or bad judgment of the dialysate filter is to be done, the control means opens the other space into the air by the opening means and suctions the fluid in the one space through the inspection passage by operating the dialysate pump, stops the dialysate pump when the pressure detecting means detects that a pressure is reduced to a predetermined first negative pressure, continues suctioning from the one space by the draining means and when the pressure detecting means detects that the pressure in the one space is reduced to a predetermined second negative pressure lower than the first negative pressure, stops an operation of the draining means; and
the judgment means monitors pressure fluctuation in the one space where the pressure is reduced to the second negative pressure by the pressure detecting means and determines that the dialysate filter is not acceptable when a pressure rise is detected.

### Advantageous Effects of Invention

According to the above described invention, by discharging the dialysate through the inspection passage connected to the one space of the dialysate filter and by opening the other space by the opening means, a differential pressure is generated between the one space and the other space, whereby leak check of the dialysate filter is conducted.

At that time, since the pressure is rapidly reduced to the first negative pressure by the dialysate pump having a large fluid feeding amount and then, the pressure is reduced to the second negative pressure by the draining means which generates a high negative pressure, a leak check can be conducted rapidly and accurately.

### Brief Description of the Drawings

[Figure 1] Figure 1 is an appearance view of a hemodialysis apparatus according to this embodiment.
[Figure 2] Figure 2 is a fluid circuit diagram of the hemodialysis apparatus.
[Figure 3] Figure 3 is a graph illustrating a state of a pressure in a dialysate circuit.
[Figure 4] Figure 4 is a fluid circuit diagram when leak check of a first dialysate filter is conducted.
[Figure 5] Figure 5 is a fluid circuit diagram when leak check of a second dialysate filter is conducted.
[Figure 6] Figure 6 is a fluid circuit diagram of the hemodialysis apparatus according to a second embodiment.

### Mode for Carrying out the Invention

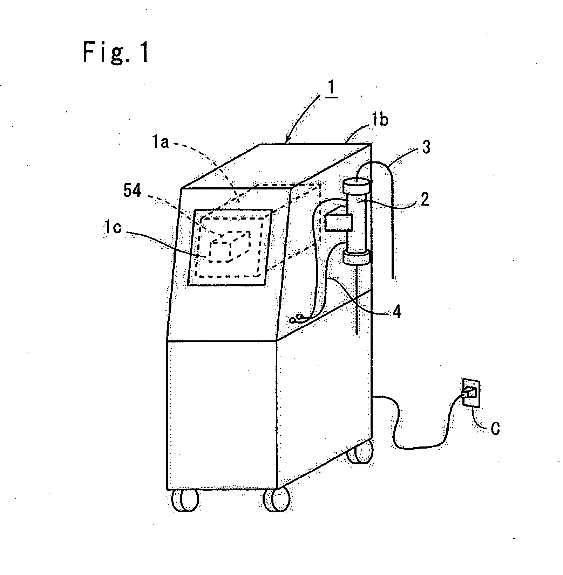

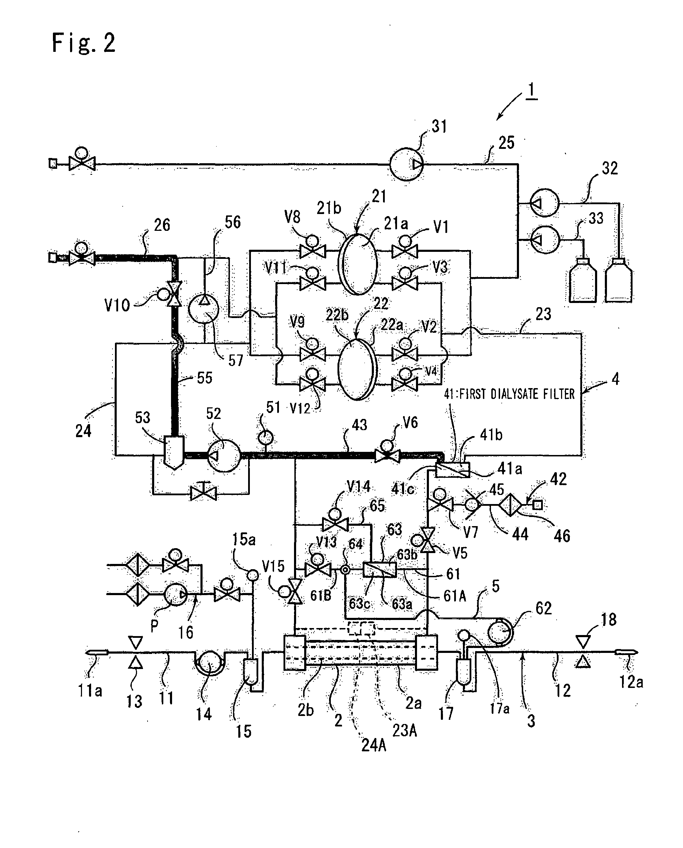

An illustrated embodiment will be described below. Figure 1 illustrates an appearance of a hemodialysis apparatus conducting dialysis treatment, and Figure 2 illustrates a fluid circuit provided inside the hemodialysis apparatus 1, and this hemodialysis apparatus 1 is operated by receiving power from a power source such as an outlet C of a hospital or the like and is also controlled by control means 1a provided therein.

The hemodialysis apparatus 1 is provided with a dialyzer 2 held outside a main body portion 1b, a blood circuit 3 connected to the dialyzer 2, a dialysate circuit 4 connected to the dialyzer 2 and provided inside the main body portion 1b, and a replenishing passage 5 for replenishing the dialysate from the dialysate circuit 4 to the blood circuit 3.

Moreover, the control means 1a is provided with a screen-display type operation panel 1c, and buttons, icons, and messages required for operations are displayed on a screen so that the operations of the device and setting of various parameters can be performed. Moreover, judgment means 54 for determining acceptability of a dialysate filter which will be described later is built in, and an alarm on the basis of a determination result and the like are also displayed.

The dialyzer 2 is composed of an exterior case 2a made of resin and a large number of hollow fibers 2b provided inside the exterior case 2a, and the inside of the hollow fiber 2b communicates with the blood circuit 3 so that blood flows therethrough and the dialysate circuit 4 communicates between the exterior case 2a and the hollow fiber 2b so that the dialysate flows in a direction opposite to that of blood.

The blood circuit 3 is composed of an artery-side passage 11 connected to a blood vessel of a patient and supplying blood to the dialyzer 2 and a vein-side passage 12 for returning the blood from the dialyzer 2 to the patient, and each of these passages is formed of a tube made of silicone.

In the artery-side passage 11, a puncture needle 11a to be pierced into the blood vessel of the patient is provided on one end thereof and the other end is connected to the dialyzer 2, and a clamp 13 for closing the artery-side passage 11, a blood pump 14 for feeding blood, and a drip chamber 15 are arranged in order from the puncture needle 11a.

The blood pump 14 is a roller pump for drawing the tube and feeding the fluid and has its operation controlled by the control means 1a so that blood can be fed from the patient to the dialyzer 2.

In the drip chamber 15, a pressure meter 15a for measuring the pressure in the drip chamber 15 and fluid-level adjusting means 16 for adjusting a fluid level in the drip chamber 15 are provided.

The fluid-level adjusting means 16 is to adjust the fluid level to a predetermined level by having air flow in by an air pump P from the state in which the drip chamber 15 is filled with a priming fluid such as saline water or the like by priming.

The vein-side passage 12 has its one end connected to the dialyzer 2, while a puncture needle 12a to be pierced into the blood vessel of the patient is provided on the other end, and a drip chamber 17 and a clamp 18 for closing the vein-side passage 12 are arranged in order from the dialyzer 2.

In the drip chamber 17, a pressure meter 17a for measuring the pressure in the drip chamber 17 is provided, and one end of the replenishing passage 5 is connected thereto, and when fresh dialysate fed through the dialysate circuit 4 is supplied to the drip chamber 17 through the replenishing passage 5, this dialysate is replenished to the patient through the vein-side passage 12.

The dialysate circuit 4 is composed of a dialysate supply passage 23 for supplying fresh dialysate to the dialyzer 2 from the first dialysate chamber 21 or the second dialysate chamber 22 and a dialysate recovery passage 24 for recovering used dialysate having passed through the dialyzer 2 to the first dialysate chamber 21 or the second dialysate chamber 22, and each of these passages is formed of a tube made of silicone.

At distal ends of the dialysate supply passage 23 and the dialysate recovery passage 24, the couplers 23A and 24A for connection to the dialyzer 2 are provided, respectively, and during washing of the dialysate circuit 4 or inspection of a dialysate filter which will be described later, as indicated by a dotted line in Figure 2, the couplers 23A and 24A are connected to each other so as to realize communication without passing through the dialyzer 2.

The first dialysate chamber 21 and the second dialysate chamber 22 have the same shape, and each of the insides thereof is divided into two chambers by a diaphragm, in which one of them is made supply chambers 21a and 22a for creating and supplying fresh dialysate, while the other is made recovery chambers 21b and 22b for recovering the used dialysate.

To each of the supply chambers 21a and 22a of the first and second dialysate chambers 21 and 22, a water supply passage 25 for supplying clean water is connected, and to each of the recovery chambers 21b and 22b, a fluid discharge passage 26 for discharging the used dialysate is connected.

In the water supply passage 25, water supply means, not shown, for supplying clean water such as RO water is connected to its upstream portion, and a downstream portion is branched into two directions, which are connected to the supply chambers 21a and 22a of the first and second dialysate chambers 21 and 22, respectively, and fluid supply valves V1 and V2 which are opened/closed by control of the control means 1a, respectively, are provided.

In the water supply passage 25, a clean water pump 31 for feeding the clean water is provided, and between this clean water pump 31 and the branch portion, an A-fluid supply means 32 for supplying an A-fluid which is a stock solution of the dialysate and a B-fluid supply means 33 for supplying a B-fluid which is a stock solution of the dialysate are connected.

In the dialysate supply passage 23, its upstream portion is branched into two directions, which are connected to the supply chambers 21a and 22a of the first and second dialysate chambers 21 and 22, respectively, while a downstream portion is connected to the dialyzer 2, and fluid supply valves V3 and V4 which are opened/closed by control of the control means 1a, respectively, are provided on the branch portion.

Moreover, in the dialysate supply passage 23, a first dialysate filter 41 for removing a hazardous component in the dialysate, opening means 42 for opening the dialysate circuit 4 into the air, and a first opening/closing valve V5 opened/closed by control of the control means 1a are provided from the branch point to the dialyzer 2.

The first dialysate filter 41 is composed of an exterior case made of resin and a filtration film 41a made of a large number of hollow fibers provided inside the exterior case similarly to the dialyzer 2.

Specifically, a space on the outside of the hollow fiber in the exterior case constitutes a space 41b on the inflow side of the dialysate to which the upstream-side portion of the dialysate supply passage 23 is connected, while a space inside the hollow fiber constitutes a space 41c on the discharge side of the dialysate to which the downstream-side portion of the dialysate supply passage 23 is connected. That is, when the dialysate passes from the outside to the inside of the hollow fiber, that is, from the inflow side to the discharge side of the dialysate, endotoxin and the like are removed, and the dialysate is purified.

The hollow fiber forming the space 41c on the discharge side of the first dialysate filter 41 has one end connected to the dialysate supply passage 23 and the other end closed by a cap.

Moreover, to the space 41b on the inflow side in the first dialysate filter 41, a first bypass passage 43 as a first inspection passage having the other end connected to the dialysate recovery passage 24 is connected, and a second opening/closing valve V6 opened/closed by control of the control means 1a is provided in the first bypass passage 43.

The first bypass passage 43 is to discharge defective dialysate in the dialysate supply passage 23 or cleaning fluid through the dialysate recovery passage 24 by opening of the second opening/closing valve V6 by the control means 1a if a defect in the dialysate is detected during dialysis treatment or if the outer surface of a hollow fiber which is the filtration film 41a is to be washed out (flushing), for example.

Moreover, if leak check of the first dialysate filter 41 which will be described later is to be conducted, similarly by opening of the second opening/closing valve V6 by the control means 1a, the fluid is discharged from the space 41b on the inflow side through the first bypass passage 43, and a differential pressure is generated inside the first dialysate filter 41.

The opening means 42 has a configuration in which, in an open passage 44 provided between the first dialysate filter 41 and the first opening/closing valve V5 in the dialysate supply passage 23, a third opening/closing valve V7 opened/closed by control of the control means 1a, a check valve 45 for preventing outflow of the dialysate, and an air filter 46 for cleaning the inflow air are provided.

If the third opening/closing valve V7 is opened, inflow of the outside air is allowed into the dialysate circuit 4 constituting a closed circuit and particularly the space 41c on the discharge side in the first dialysate filter 41 and a space 63b on the inflow side in a second dialysate filter 63 which will be described later are opened to the air.

At that time, the air flowing into the dialysate circuit 4 by opening into the air is cleaned by the air filter 46, and the check valve 45 prevents outflow of the dialysate.

In the dialysate recovery passage 24, an upstream portion is connected to the dialyzer 2, and a downstream portion is branched into two directions, which are connected to the recovery chambers 21b and 22b of the first and second dialysate chambers 21 and 22, respectively, and in this branch portions, recovery valves V8 and V9 opened/closed by control of the control means 1a are provided, respectively.

Moreover, in the dialysate recovery passage 24, a pressure sensor 51 for measuring a pressure in the dialysate circuit 4 and a dialysate pump 52 for feeding the dialysate are provided, and between the dialysate pump 52 and the first and second dialysate chambers 21 and 22, a deaeration tank 53 for removing air bubbles in the dialysate flowing through the dialysate recovery passage 24 is provided.

When the dialysate pump 52 is operated, the fluid on the upstream side of the dialysate pump 52 in the dialysate recovery passage 24 is suctioned and discharged on the downstream side, but in a state in which the fluid is not supplied to this dialysate recovery passage 24, the upstream side of the dialysate pump 52 can be brought into a negative pressure.

However, the dialysate pump 52 is composed of a so-called gear pump and has characteristics that, though its fluid feeding amount is high, since the fluid is fed by meshed two gears, they are worn in a state without liquid and cannot be used or since air feeding is not its original purpose, its airtightness is low, and the inside of the dialysate circuit 4 cannot be brought to a high negative pressure.

The deaeration tank 53 is to remove air bubbles generated from the fed dialysate, and to this deaeration tank 53, a second bypass passage 55 as a branch passage communicating with the fluid discharge passage 26 is connected, and a fourth opening/closing valve V10 opened/closed by control of the control means 1a is provided in the second bypass passage 55.

In the fluid discharge passage 26, its upstream portion is branched into two directions, which are connected to the recovery chambers 21b and 22b of the first and second dialysate chambers 21 and 22, respectively, while the downstream portion is connected to a fluid discharge pipe, not shown, installed in a medical institution, and fluid discharge valves V11 and V12 opened/closed by control of the control means 1a, respectively, are provided in the branch portion.

To the dialysate recovery passage 24, a drainage passage 56 communicating with the fluid discharge passage 26 is connected, and its upstream portion is connected between the dialysate pump 52 in the dialysate recovery passage 24 and a branch point, while the downstream portion is connected to the downstream side of the branch point in the fluid discharge passage 26.

Moreover, a drainage pump 57 for performing ultrafiltration by discharging the used dialysate from the dialysate recovery passage 24 is provided in the drainage passage 56.

The drainage pump 57 is a so-called plunger pump and is configured to repeat suction into a cylinder and discharge, and though a fluid feeding amount can be set precisely and a high negative pressure can be generated in the dialysate circuit 4, the fluid feeding amount is low.

The replenishing passage 5 is disposed between a connection passage 61 disposed between the dialysate supply passage 23 and the dialysate recovery passage 24 and the drip chamber 17 of the vein-side passage 12 in the blood circuit 3 and in the replenishing passage 5, a replenishing pump 62 formed of a tube pump substantially similar to the blood pump 14 is provided.

An end portion on the dialysate supply passage 23 side in the connection passage 61 is connected between the first opening/closing valve V5 and the dialyzer 2, and an end portion on the dialysate recovery passage 24 side is connected between the dialyzer 2 in the dialysate recovery passage 24 and the pressure sensor 51.

Moreover, in the connection passage 61, a second dialysate filter 63 for removing a hazardous component in the dialysate, a connection port 64 for detachably connecting the replenishing passage 5, and a fifth opening/closing valve V13 controlled by the control means 1a are provided in order from the dialysate supply passage 23.

The second dialysate filter 63 is divided by a filtration film 63a made of a hollow fiber similarly to the first dialysate filter 41 into a space 63b on the inflow side which is the outside of the hollow fiber in the exterior case and a space 63c on the discharge side made of the inside of the hollow fiber so that the dialysate flowing from the connection passage 61 flows out to the inside of the hollow fiber, and in the case of replenishment, the dialysate purified by the first dialysate filter 41 is further purified and then, the dialysate is supplied to the blood circuit 3 through the replenishing passage 5.

Here, the space 63b on the inflow side of the second dialysate filter 63 is connected to the dialysate supply passage 23 through an inflow section 61A of the connection passage 61 and communicates with the space 41C on the discharge side of the first dialysate filter 41.

Moreover, a discharge section 61B having the space 63c on the discharge side of the second dialysate filter 63 communicate with the dialysate recovery passage 24 in the connection passage 61 is used as a second inspection passage for conducting leak check of the second dialysate filter 63, and when leak check is to be conducted, the dialysate is discharged to the dialysate recovery passage 24 through the space 63c on the discharge side.

Moreover, to the space 63b on the inflow side in the second dialysate filter 63, a third bypass passage 65 for performing washing (flushing) of the filtration film 63a is connected, and the other end of this third bypass passage 65 is connected to the dialysate recovery passage 24, and a sixth opening/closing valve V14 controlled by the control means 1a is also provided.

Furthermore, in the dialysate recovery passage 24, a seventh opening/closing valve V15 controlled by the control means 1a is provided on the dialyzer 2 side (the coupler 24A side) of a connection position of the connection passage 61.

A method of use of the hemodialysis apparatus 1 having the above described configuration will be described below by using Figures 2 to 5. Here, before conducting the dialysis treatment, a method of operation when leak check is conducted for checking leakage of the filtrate films of the first and second dialysate filters 41 and 63 will be described.

In the hemodialysis apparatus 1, the dialysate circuit 4 is filled with rinse fluid generated by washing after the previous treatment, and the dialyzer 2, the blood circuit 4, and the replenishing passage 5 are not connected, and the dialysate supply passage 23 and the dialysate recovery passage 24 are made to communicate with each other by the couplers 23A and 24A.

The control means 1a closes the first opening/closing valve V5 of the dialysate supply passage 23 in a state in which all the fluid supply valves V1, V2, the supply valves V3, V4, the recovery valves V8 and V9, and the fluid discharge valves V11 and V12 are closed, opens the second opening/closing valve V6 of the first bypass passage 43, opens the fourth opening/closing valve V10 of the second bypass passage 55, and closes the fifth opening/closing valve V13 of the connection passage 61.

In the following description, the sixth opening/closing valve V14 of the third bypass passage 65 and the seventh opening/closing valve V15 of the dialysate recovery passage 24 are supposed to be closed all the time.

Moreover, the control means 1a opens the third opening/closing valve V7 in the opening means 42, whereby the space 41c on the discharge side of the first dialysate filter 41 communicating with the opening means 42 is opened to the air.

By operating the dialysate pump 52 in this state, the fluid in the dialysate circuit 4 is discharged along a path indicated by a bold line in Figure 2, the fluid is suctioned from the space 41b on the inflow side of the first dialysate filter 41, and the fluid is suctioned also from the space 41c on the discharge side through the filtration film 41a.

However, since air cannot pass the filtration film 41a, when the dialysate pump 52 further tries to suction the fluid from the space 41b on the inflow side, a differential pressure between the negative pressure of the space 41b on the inflow side and the atmospheric pressure of the space 41c on the discharge side increases.

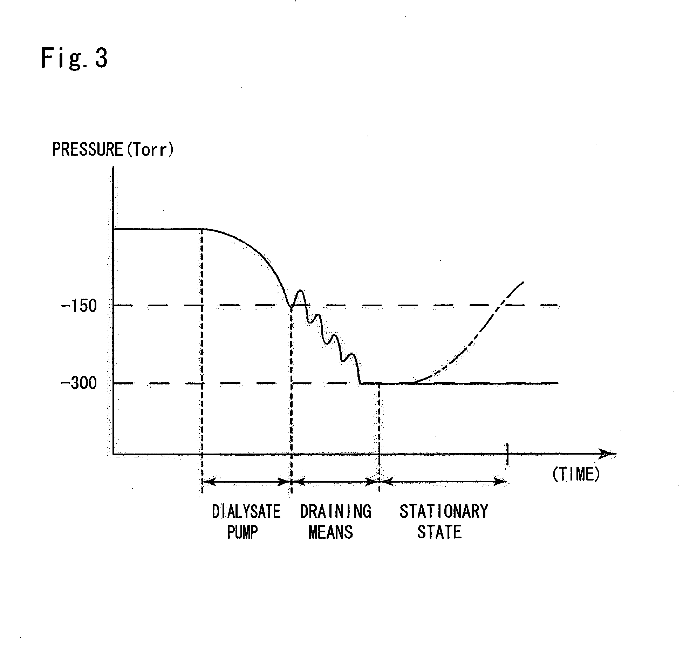

Figure 3 is a graph illustrating a change of the pressure of the dialysate detected by the pressure sensor 51 provided in the dialysate recovery passage 24, in which the lateral axis indicates time and the vertical axis indicates the pressure of the dialysate.

As illustrated in Figure 3, when the fluid is fed by the dialysate pump 52, the inside of the dialysate circuit 4 can be brought into a some degree of negative pressure, but the gear pump constituting the dialysate pump 52 cannot be used in a state where the fluid does not communicate, and moreover, a low negative pressure cannot be generated.

Thus, if the pressure in the dialysate recovery passage 24 is reduced to a predetermined first negative pressure (-150 Torr (-20kPa), for example) by the dialysate pump 52 and this is detected by the pressure sensor 51, the control means 1a stops the dialysate pump 52 and then, operates the drainage pump 57 of the drainage passage 56.

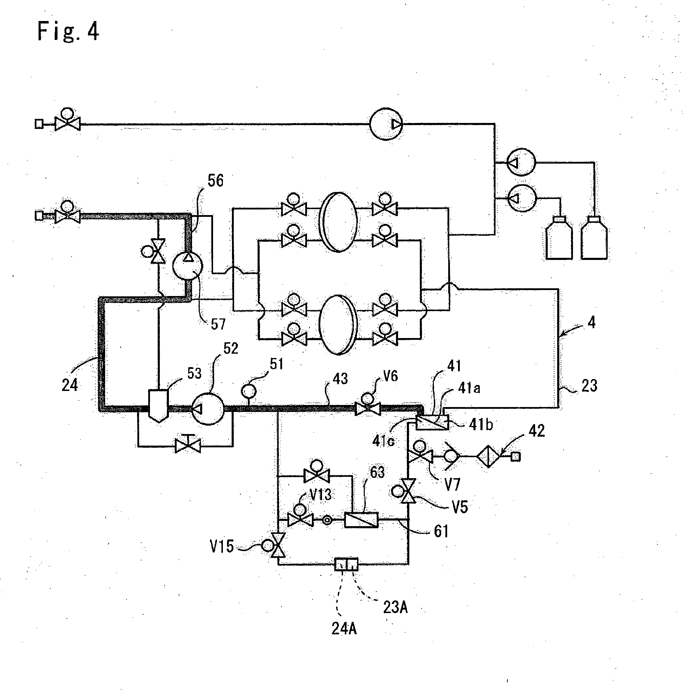

As illustrated in Figure 4, if the drainage pump 57 discharges the remaining fluid to the fluid discharge passage 26 through the drainage passage 56, the space 41b is further suctioned. As described above, since the drainage pump 57 can generate a lower negative pressure, the differential pressure between the negative pressure of the space 41b on the inflow side and the atmospheric pressure of the space 41c on the discharge side in the first dialysate filter 41 can be further increased.

As a result, the negative pressure in the dialysate circuit 4 can be reduced to a predetermined second negative pressure (-300 Torr (-40kPa), for example) lower than the first negative pressure as illustrated in Figure 3, and if the pressure sensor 51 detects the second negative pressure, the control means 1a stops the drainage pump 57.

The control means 1a maintains a stationary state in which the dialysate pump 52 and the drainage pump 57 are stopped as above and no fluid communicates for a predetermined time and monitors pressure fluctuation during that period.

Then, in the stationary period, if the second negative pressure is maintained, the judgment means 54 considers that air does not leak to the space 41b on the inflow side from the space 41c on the discharge side in the filtration film 41a and determines that no leakage occurs in the first dialysate filter 41.

On the other hand, as indicated by a virtual line, if pressure fluctuation such that the second negative pressure rises to the positive pressure side is detected in the stationary period, the judgment means 54 considers that air leaks from the space 41c on the discharge side to the space 41b on the inflow side in the filtration film 41a and determines that there is leakage in the first dialysate filter 41.

Then, the control means 1a displays such leak check result of the first dialysate filter 41 on the operation panel 1c and generates a predetermined alarm if leakage is detected.

When the leak check of the first dialysate filter 41 is finished as above, the leak check of the second dialysate filter 63 is conducted continuously by control of the control means 1a.

The control means 1a opens the first opening/closing valve V5 of the dialysate supply passage 23, closes the second opening/closing valve V6 of the first bypass passage 43, and opens the fifth opening/closing valve V13 of the connection passage 61.

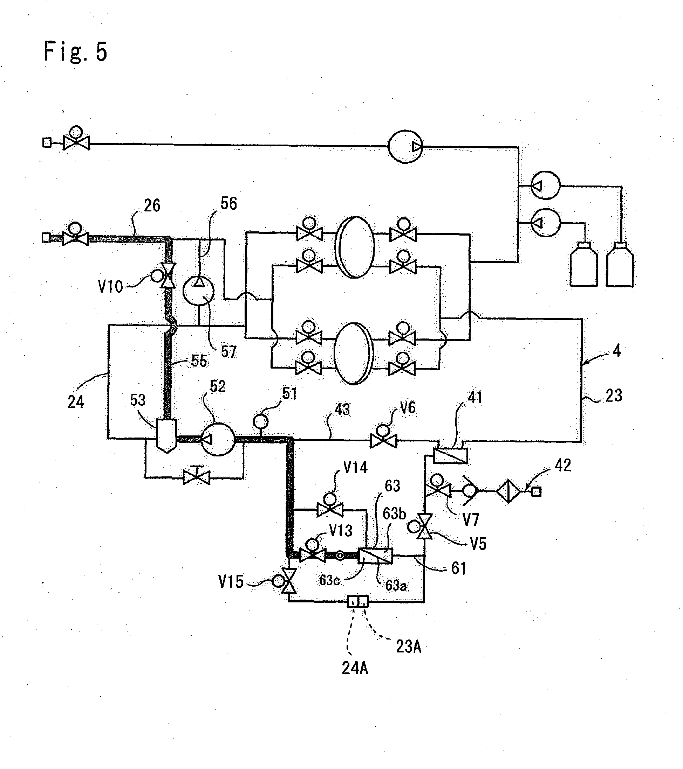

When the control means 1a operates the dialysate pump 52 in this state, the fluid is discharged along the path indicated in Figure 5, the fluid is suctioned from the space 63c on the outflow side of the second dialysate filter 63, and the fluid is also suctioned from the space 63b on the inflow side through the filtration film 63a.

However, since air cannot pass through the filtration film 63a, when the dialysate pump 52 further tries to suction the fluid from the space 63c on the discharge side, a differential pressure between the atmospheric pressure of the space 63b on the inflow side and the negative pressure of the space 63c on the discharge side increases.

After that, the control means 1a stops the dialysate pump 52 at a point of time when the pressure in the dialysate recovery passage 24 is reduced to the first negative pressure similarly to the leak check of the first dialysate filter 41, operates the drainage pump 57, and maintains the stationary state by stopping the drainage pump 57 if the pressure is reduced to the second negative pressure.

Then, the judgment means 54 provided on the control means 1a monitors pressure fluctuation in the dialysate recovery passage 24 and determines presence of leakage in the second dialysate filter 63.

According to the above described embodiment, the differential pressure is generated between the spaces 41b and 63b on the inflow side and the spaces 41c and 63c on the discharge side in order to conduct leak check of the first and second dialysate filters 41 and 63, but at that time, accurate and rapid determination can be made by reducing the pressure to the first negative pressure by the dialysate pump 52 and by reducing the pressure to the second negative pressure by the drainage pump 57.

That is, even though the dialysate pump 52 cannot generate a sufficient negative pressure, since the dialysate feeding capacity is high, the pressure can be reduced more rapidly than pressure reduction to the second negative pressure only by the drainage pump 57.

On the other hand, even though the drainage pump 57 has low fluid feeding capacity, since it can generate a sufficient negative pressure, a sufficient differential pressure can be generated sandwiching the filtration films 41a and 63a in the first and second dialysate filters 41 and 63 by reducing the pressure in the dialysate recovery passage 24 to the second negative pressure, and leak check can be conducted more reliably than the case of generating the negative pressure only by the dialysate pump 52.

Moreover, in the case of this embodiment, the opening means 42 is provided between the first and second dialysate filters 41 and 63 in the dialysate supply passage 23, whereby opening to the air performed at leak check of the first and second dialysate filters 41 and 63 can also be done by the single opening means 42.

That is, in the second dialysate filter 63, for example, in the above described embodiment, it can be also configured such that the space 63c on the discharge side can be opened to the air similarly to the leak check of the first dialysate filter 41, but in that case, opening means needs to be provided separately in the dialysate recovery passage 24, for example.

The hemodialysis apparatus 1 in the above described embodiment is a so-called dialyzer for personal use but it may be a so-called monitoring device for dialysis performing hemodialysis using the dialysate supplied from the dialysate supply device or may be provided with draining means different from the plunger pump.

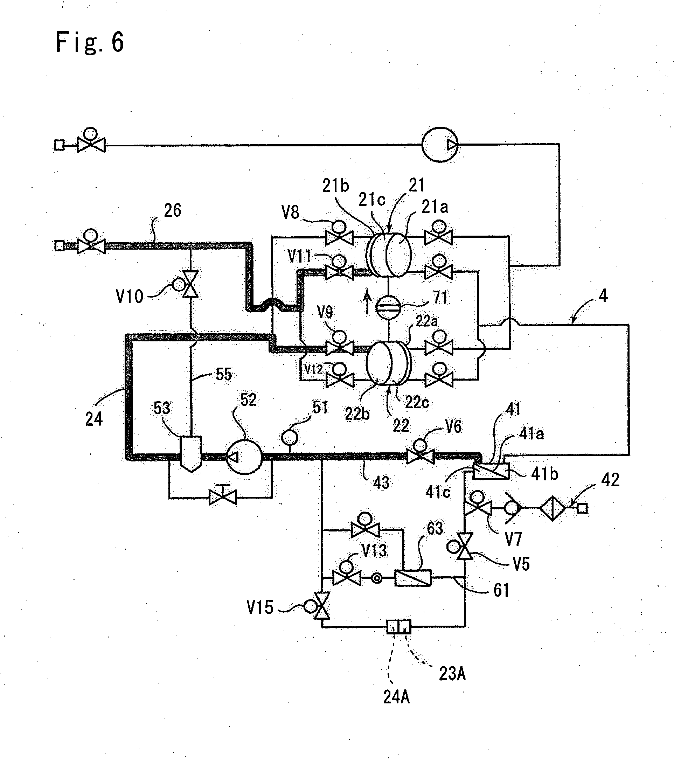

Specifically speaking, Figure 6 illustrates the hemodialysis apparatus 1 according to a second embodiment, and in the dialysate circuit 4 in this hemodialysis apparatus 1, the first and second dialysate chambers 21 and 22 divided into three chambers by two diaphragms, respectively, are provided, and in these first and second dialysate chambers 21 and 22, the supply chambers 21a and 22a for supplying the fresh dialysate, the recovery chambers 21b and 22b for recovering the used dialysate, and intermediate chambers 21c and 22c in which silicone oil is filed are formed, and a silicone oil pump 71 as draining means for feeding silicone oil between each of the intermediate chambers 21c and 22c is provided between the intermediate chamber 21c and the intermediate chamber 22c.

If the silicone oil pump 71 feeds silicone oil from the intermediate chamber 21c of the first dialysate chamber 21 to the intermediate chamber 22c of the second dialysate chamber 22, for example, the volume of the intermediate chamber 21c is reduced and the volume of the recovery chamber 21b increases by that portion and thus, whereby the pressure of the dialysate recovery passage 24 is reduced, and drainage is performed in the dialyzer 2 from blood to the dialysate.

Moreover, when leak check of the first and second dialysate filters 41 and 63 is to be conducted in the hemodialysis apparatus 1 provided with draining means having the above configuration, first, similarly to the above described first embodiment, the fluid is suctioned from the space 41b on the inflow side and the space 41c on the outflow side of the first dialysate filter 41 by using the dialysate pump 52, and the pressure inside of the dialysate recovery passage 24 is reduced to the first negative pressure.

From this state, in the example illustrated in Figure 6, the recovery valve V9 of the second dialysate chamber 22 and the fluid discharge valve V11 of the first dialysate chamber 21 are opened, the silicone oil pump 71 is operated, the silicone oil is moved from the intermediate chamber 22c to the intermediate chamber 21c so as to further reduce the pressure in the dialysate recovery passage 24 to the second negative pressure lower than the first negative pressure.

Then, similarly to the above described embodiment, the judgment means 54 of the control means 1a determines presence of leakage of the first dialysate filter 41. Moreover, similarly to the above described first embodiment and the first dialysate filter 41 in this embodiment, presence of leakage of the second dialysate filter 63 can be determined.

In the above described embodiment, after the dialysate pump 52 is stopped, the drainage pump 57 or the silicone oil pump 71 is operated, but they may be operated while the dialysate pump 52 is still operating.

### Reference Signs List

- 1: hemodialysis apparatus
- 1a: control means
- 2: dialyzer
- 3: blood circuit
- 4: dialysate circuit
- 5: replenishing passage
- 11: artery-side passage
- 12: vein-side passage
- 23: dialysate supply passage
- 24: dialysate recovery passage
- 41: first dialysate filter
- 41a: filtration film
- 41b: space on the inflow side
- 41c: space on the discharge side
- 42: opening means
- 43: first bypass passage (first inspection passage)
- 51: pressure sensor (pressure detecting means)
- 52: dialysate pump
- 54: judgment means
- 57: drainage pump
- 61: connection passage
- 61B: discharge section (second inspection passage)
- 63: second dialysate filter
- 63a: filtration film
- 63b: space on the inflow side
- 63c: space on the discharge side
- 71: silicone oil pump

## Claims

1. A hemodialysis apparatus (1) that is provided with a dialyzer (2) which performs hemodialysis, a dialysate circuit (4) composed of a dialysate supply passage (23) for supplying fresh dialysate to the dialyzer (2) and a dialysate recovery passage (24) for recovering used dialysate having passed through the dialyzer (2), a dialysate pump (52) provided in the dialysate recovery passage (24) and feeding the used dialysate, draining means for drainage from blood through the dialyzer (2), a dialysate filter (41, 63) having an inside divided by a filtration film (41a, 63a) into a space on the inflow side (41 b, 63b) and a space on the discharge side (41 c, 63c) and purifying and discharging the fresh dialysate flowing from the dialysate supply passage (23), and control means (1 a) for controlling operations of the dialysate pump (52) and the draining means, comprising:
an inspection passage (43, 61 B) for connecting one of the space on the inflow side (41b) or the space on the discharge side (63c) of the dialysate filter (41, 63) to the dialysate recovery passage (24); opening means (42) for opening the other space on the side opposite to the one space of the dialysate filter (41, 63) into the air; pressure detecting means (51) for detecting pressure fluctuation in the one space; and judgment means (54) for good or bad judgment of the dialysate filter (41, 63) on the basis of a detection result of the pressure detecting means (51),
**characterised in that** when good or bad judgment of the dialysate filter (41, 63) is to be done, the control means (1 a) opens the other space into the air by the opening means (42) and suctions the fluid in the one space through the inspection passage (43, 61 B) by operating the dialysate pump (52), stops the dialysate pump (52) when the pressure detecting means (51) detects that a pressure is reduced to a predetermined first negative pressure, continues suctioning from the one space by the draining means and when the pressure detecting means (51) detects that the pressure in the one space is reduced to a predetermined second negative pressure lower than the first negative pressure, stops an operation of the draining means; and
the judgment means (54) monitors pressure fluctuation in the one space where the pressure is reduced to the second negative pressure by the pressure detecting means (51) and determines that the dialysate filter (41, 63) is not acceptable when a pressure rise is detected.

2. The hemodialysis apparatus (1) according to claim 1, further comprising:
a first filter (41) located on the upstream side and a second filter (63) located on a downstream side as the dialysate filter, wherein
a space on the inflow side (41 b) of the first filter (41) and the dialysate recovery passage (24) are connected by a first inspection passage, a space on the discharge side (63c) of the second filter (63) and the dialysate recovery passage (24) are connected by a second inspection passage (61 B), and a space on the discharge side of the first filter (41 c) and a space on the inflow side of the second filter (63b) are made to communicate by the dialysate supply passage (23); and
the opening means (42) is provided in the dialysate supply passage (23) between the first filter (41) and the second filter (63).

3. The hemodialysis apparatus (1) according to claim 1 or 2, further comprising:
a dialysate chamber (21, 22) for recovering the used dialysate from the dialysate recovery passage (24) and a fluid discharge passage (26) for discharging the used dialysate from the dialysate chamber (21, 22), wherein
a branch passage (55) communicating with the fluid discharge passage (26) is provided between the dialysate pump (52) in the dialysate recovery passage (24) and the dialysate chamber (21, 22); and
when good or bad judgment of the dialysate filter (41, 63) is to be done, the fluid suctioned by the dialysate pump (52) is discharged from the branch passage (55) to the fluid discharge passage (26).

4. The hemodialysis apparatus (1) according to any one of claims 1 to 3, wherein
two dialysate chambers (21, 22) for supplying/discharging the dialysate are provided, the inside of each of the dialysate chambers being divided into a supply chamber (21 a, 22a) for supplying fresh dialysate, a recovery chamber (21 b, 22b) for recovering used dialysate, and an intermediate chamber (21 c, 22c) formed between the supply chamber (21 a, 22a) and the recovery chamber (21 b, 22b) and in which silicone oil is filled;
a silicone oil pump (71) is further provided for feeding silicone oil between each of the intermediate chambers (21c ,22c) in the two dialysate chambers (21, 22); and
as the draining means, a volume of the intermediate chamber (21 c, 22c) is changed by the silicone oil pump (71) and drained.

5. The hemodialysis apparatus (1) according to any one of claims 1 to 3, further comprising:
a drainage pump (57) connecting a drainage passage (56) to the dialysate recovery passage (24) and discharging the used dialysate to the drainage passage (56), wherein
as the draining means, drainage is performed by the drainage pump (57).

## Patentansprüche

1. Hämodialysator (1), der mit einem Dialysator (2), der eine Hämodialyse ausführt, einem Dialysat-Kreis (4), der aus einem Dialysat-Zufuhr-Durchlass (23) für das Versorgen des Dialysators (2) mit frischem Dialysat, und einem Dialysat-Rückgewinnungs-Durchlass (24) für die Rückgewinnung von verwendetem Dialysat besteht, das den Dialysator (2) durchlaufen hat, einer Dialysator-Pumpe (52), die im Dialysat-Rückgewinnungs-Durchlass (24) bereitgestellt ist und das verwendete Dialysat zuführt, einem Drainage-Mittel für die Drainage aus Blut durch den Dialysator (2) hindurch, einem Dialysat-Filter (41, 63), der ein durch einen Filtrationsfilm (41 a, 63a) in einen Raum auf der Zufluss-Seite (41 b, 63b) und einen Raum auf der Abfluss-Seite (41 c, 63c) unterteiltes Inneres aufweist und der das vom Dialysat-Zufuhr-Durchlass (23) fließende frische Dialysat reinigt und abführt, und einem Steuerungsmittel (1a) für das Steuern der Funktionsweise der Dialysat-Pumpe (52) und des Drainage-Mittels, versehen ist, umfassend:
einen Kontroll-Durchlass (43, 61 B) zum Verbinden von einem des Raumes auf der Zufluss-Seite (41 b) oder des Raumes auf der Abfluss-Seite (63c) des Dialysat-Filters (41, 63) mit dem Dialysat-Rückgewinnungs-Durchlass (24); ein Öffnungsmittel (42) für das Öffnen des anderen Raumes auf derjenigen Seite, die dem einen Raum des Dialysat-Filters (41, 63) entgegengesetzt ist, nach außen; ein Druck-Detektionsmittel (51) für das Detektieren von Druckschwankungen in dem einen Raum; und ein Beurteilungsmittel (54) für eine gute oder schlechte Beurteilung des Dialysat-Filters (41, 63) auf Basis eines Detektionsergebnisses des Druck-Detektionsmittels (51),
**dadurch gekennzeichnet, dass**, wenn eine gute oder schlechte Beurteilung des Dialysat-Filters (41, 63) vorgenommen werden soll, das Steuerungsmittel (1a) den anderen Raum nach außen durch das Öffnungsmittel (42) öffnet und das Fluid in dem einen Raum durch den Kontroll-Durchlass (43, 61 B) hindurch durch ein Betreiben der Dialysat-Pumpe (52) absaugt, die Dialysat-Pumpe (52) anhält, wenn das Druck-Detektionsmittel (51) detektiert, dass ein Druck auf einen vorbestimmten ersten negativen Druck reduziert ist, mit dem Absaugen aus dem einen Raum durch das Drainage-Mittel fortfährt und, wenn das Druck-Detektionsmittel (51) detektiert, dass der Druck in dem einen Raum auf einen vorbestimmten zweiten negativen Druck reduziert ist, der niedriger als der erste negative Druck ist, einen Arbeitsvorgang des Drainage-Mittels beendet; und
das Beurteilungsmittel (54) Druckschwankungen in dem einen Raum, in dem der Druck auf den zweiten negativen Druck durch das Druck-Detektionsmittel (51) reduziert ist, überwacht und bestimmt, dass der Dialysat-Filter (41, 63) nicht zulässig ist, wenn ein Druckanstieg detektiert wird.

2. Hämodialysator (1) gemäß Anspruch 1, ferner umfassend:
einen ersten Filter (41), der auf der Stromaufwärts-Seite angeordnet ist, und einen zweiten Filter (63), der auf einer Stromabwärts-Seite wie der Dialysat-Filter angeordnet ist, worin
ein Raum auf der Zufluss-Seite (41 b) des ersten Filters (41) und der Dialysat-Rückgewinnungs-Durchlass (24) durch einen ersten Kontroll-Durchlass verbunden sind, ein Raum auf der Abfluss-Seite (63c) des zweiten Filters (63) und der Dialysat-Rückgewinnungs-Durchlass (24) durch einen zweiten Kontroll-Durchlass (61 B) verbunden sind, und ein Raum auf der Abfluss-Seite des ersten Filters (41 c) und ein Raum auf der Zufluss-Seite des zweiten Filters (63b) zur Kommunikation durch den Dialysat-Zufuhr-Durchlass (23) ausgebildet sind; und
das Öffnungsmittel (42) im Dialysat-Zufuhr-Durchlass (23) zwischen dem ersten Filter (41) und dem zweiten Filter (63) bereitgestellt ist.

3. Hämodialysator (1) nach Anspruch 1 oder 2, ferner umfassend:
eine Dialysat-Kammer (21, 22) für die Rückgewinnung des verwendeten Dialysats aus dem Dialysat-Rückgewinnungs-Durchlass (24), und einen Fluid-Abfluss-Durchlass (26) für das Abführen des verwendeten Dialysats aus der Dialysat-Kammer (21, 22), worin
ein mit dem Fluid-Abfluss-Durchlass (26) kommunizierender Nebendurchlass (55) zwischen der Dialysat-Pumpe (52) im Dialysat-Rückgewinnungs-Durchlass (24) und in der Dialysat-Kammer (21, 22) bereitgestellt ist; und
das durch die Dialysat-Pumpe (52) abgesaugte Fluid aus dem Nebendurchlass (55) in den Fluid-Abfluss-Durchlass (26) abgeführt wird, wenn eine gute oder schlechte Beurteilung des Dialysat-Filters (41, 63) vorgenommen werden soll.

4. Hämodialysator (1) gemäß einem der Ansprüche 1 bis 3, worin
zwei Dialysat-Kammern (21, 22) für das Zuführen/Abführen des Dialysats bereitgestellt werden, wobei das Innere von jeder der Dialysat-Kammern in eine Zufuhr-Kammer (21 a, 22a) zur Zufuhr von frischem Dialysat, eine Rückgewinnungskammer (21 b, 22b) zur Rückgewinnung von verwendetem Dialysat, und eine Zwischenkammer (21 c, 22c) unterteilt wird, die zwischen der Zufuhrkammer (21 a, 22a) und der Rückgewinnungsskammer (21 b, 22b) ausgebildet ist und die mit Silikonöl gefüllt wird;
eine Silikonölpumpe (71) ferner für das Zuführen von Silikonöl zwischen jede der Zwischenkammern (21 c, 22c) in den zwei Dialysat-Kammern (21, 22) bereitgestellt wird; und
als Drainage-Mittel, ein Volumen der Zwischenkammer (21 c, 22c) durch die Silikonölpumpe (71) verändert, und abgeleitet wird.

5. Hämodialysator (1) gemäß einem der Ansprüche 1 bis 3, ferner umfassend:
eine Drainagepumpe (57), die einen Drainage-Durchlass (56) mit dem Dialysat-Rückgewinnungs-Durchlass (24) verbindet, und das verwendete Dialysat in den Drainage-Durchlass (56) abführt, worin
als Drainage-Mittel, die Drainage durch die Drainagepumpe (57) ausgeführt wird.

## Revendications

1. Appareil d'hémodialyse (1) qui est pourvu d'un dialyseur (2) qui effectue une hémodialyse, d'un circuit de dialysat (4) composé d'un passage de fourniture de dialysat (23) pour fournir du dialysat frais au dialyseur (2) et d'un passage de récupération de dialysat (24) pour récupérer le dialysat utilisé qui est passé à travers le dialyseur (2), d'une pompe à dialysat (52) prévue dans le passage de récupération de dialysat (24) et fournissant le dialysat utilisé, de moyens de drainage pour le drainage à partir du sang à travers le dialyseur (2), d'un filtre à dialysat (41, 63) comportant une partie intérieure divisée par un film de filtration (41a, 63a) en un espace du côté flux d'entrée (41b, 63b) et un espace du côté de décharge (41c, 63c) et purifiant et déchargeant le dialysat frais circulant à partir du passage de fourniture de dialysat (23), et de moyens de commande (la) pour commander les opérations de la pompe à dialysat (52) et des moyens de drainage, comprenant :
un passage d'inspection (43, 61B) pour relier l'un de l'espace du côté flux d'entrée (41b) ou de l'espace du côté de décharge (63c) du filtre à dialysat (41, 63) au passage de récupération de dialysat (24) ; des moyens d'ouverture (42) pour ouvrir l'autre espace du côté opposé au dit un espace du filtre à dialysat (41, 63) à l'air ; des moyens de détection de pression (51) pour détecter une fluctuation de pression dans ledit un espace ; et des moyens de jugement (54) pour porter un jugement bon ou mauvais du filtre à dialysat (41, 63) sur la base d'un résultat de détection des moyens de détection de pression (51),
**caractérisé en ce que**, lorsqu'un jugement bon ou mauvais du filtre à dialysat (41, 63) doit être porté, les moyens de commande (la) ouvrent l'autre espace à l'air par les moyens d'ouverture (42) et aspirent le fluide dans ledit un espace à travers le passage d'inspection (43, 61B) en mettant en oeuvre la pompe à dialysat (52), arrêtent la pompe à dialysat (52) lorsque les moyens de détection de pression (51) détectent qu'une pression est réduite à une première pression négative prédéterminée, poursuivent l'aspiration à partir dudit un espace par les moyens de drainage et, lorsque les moyens de détection de pression (51) détectent que la pression dans ledit un espace est réduite à une deuxième pression négative prédéterminée inférieure à la première pression négative, arrêtent le fonctionnement des moyens de drainage ; et
les moyens de jugement (54) surveillent la fluctuation de pression dans ledit un espace où la pression est réduite à la deuxième pression négative par les moyens de détection de pression (51) et déterminent que le filtre à dialysat (41, 63) n'est pas acceptable lorsqu'une augmentation de pression est détectée.

2. Appareil d'hémodialyse (1) selon la revendication 1, comprenant en outre :
un premier filtre (41) situé du côté amont et un deuxième filtre (63) situé d'un côté aval en tant que filtre à dialysat, dans lequel
un espace du côté flux d'entrée (41b) du premier filtre (41) et le passage de récupération de dialysat (24) sont reliés par un premier passage d'inspection, un espace du côté de décharge (63c) du deuxième filtre (63) et le passage de récupération de dialysat (24) sont reliés par un deuxième passage d'inspection (61B), et un espace du côté de décharge du premier filtre (41c) et un espace du côté flux d'entrée du deuxième filtre (63b) sont mis en communication par le passage de fourniture de dialysat (23) ; et
les moyens d'ouverture (42) sont prévus dans le passage de fourniture de dialysat (23) entre le premier filtre (41) et le deuxième filtre (63).

3. Appareil d'hémodialyse (1) selon la revendication 1 ou 2, comprenant en outre :
une chambre de dialysat (21, 22) pour récupérer le dialysat utilisé à partir du passage de récupération de dialysat (24) et un passage de décharge de fluide (26) pour décharger le dialysat utilisé de la chambre de dialysat (21, 22), dans lequel
un passage de branchement (55) communiquant avec le passage de décharge de fluide (26) est prévu entre la pompe à dialysat (52) dans le passage de récupération de dialysat (24) et la chambre de dialysat (21, 22) ; et
lorsqu'un jugement bon ou mauvais du filtre à dialysat (41, 63) doit être porté, le fluide aspiré par la pompe à dialysat (52) est déchargé du passage de branchement (55) vers le passage de décharge de fluide (26).

4. Appareil d'hémodialyse (1) selon l'une quelconque des revendications 1 à 3, dans lequel
deux chambres de dialysat (21, 22) pour fournir/décharger le dialysat sont prévues, l'intérieur de chacune des chambres de dialysat étant divisé en une chambre de fourniture (21a, 22a) pour fournir du dialysat frais, une chambre de récupération (21b, 22b) pour récupérer le dialysat utilisé, et une chambre intermédiaire (21c, 22c) formée entre la chambre de fourniture (21a, 22a) et la chambre de récupération (21b, 22b) et qui est remplie d'une huile de silicone ;
une pompe à huile de silicone (71) est en outre prévue pour fournir de l'huile de silicone entre chacune des chambres intermédiaires (21c, 22c) dans les deux chambres de dialysat (21, 22) ; et
en tant que moyens de drainage, un volume de la chambre intermédiaire (21c, 22c) est modifié par la pompe à huile de silicone (71) et vidé.

5. Appareil d'hémodialyse (1) selon l'une quelconque des revendications 1 à 3, comprenant en outre :
une pompe de drainage (57) reliant un passage de drainage (56) au passage de récupération de dialysat (24) et déchargeant le dialysat utilisé vers le passage de drainage (56), dans lequel
en tant que moyens de drainage, le drainage est effectué par la pompe de drainage (57).
